Europäisches Patentamt

**European Patent Office**

**Office européen des brevets**

(19)

(11) Publication number: **0 073 617**
A1

(12) # EUROPEAN PATENT APPLICATION

(21) Application number: **82304404.5**

(22) Date of filing: **20.08.82**

(51) Int. Cl.³: **A 61 C 1/08, A 61 B 17/36**

(30) Priority: **25.08.81 GB 8125944**

(43) Date of publication of application: **09.03.83**
**Bulletin 83/10**

(84) Designated Contracting States: **AT BE CH DE FR GB IT LI LU NL SE**

(71) Applicant: **Pembery, Peter John, 18 Summersbury Drive Shalford, Guildford Surrey (GB)**

(72) Inventor: **Pembery, Peter John, 18 Summersbury Drive Shalford, Guildford Surrey (GB)**

(74) Representative: **Feakins, Graham Allan et al, Haseltine Lake & Co. Hazlitt House 28, Southampton Buildings Chancery Lane, London WC2A 1AT (GB)**

(54) **Laser dental handpiece.**

(57) A dental handpiece arranged to perform dental work in the mouth by means of laser energy, the handpiece including a bundle (2) of optical fibres (3) leading to a face (5A) of a head (5). A water/air supply tube (4) leads to an outlet (6) adjacent to the head. In use, the laser beams are focussed at a point (9) where the dental work is performed, the point (9) being adjustable relatively to the head (5) and being able to be de-focussed. A probe (8) is provided to guide the operator during use.

1

## LASER DENTAL HANDPIECE

According to the present invention, there is provided a dental handpiece arranged to perform dental work in the mouth by means of a laser beam.

For a better understanding of the invention and to show how the same may be carried into effect, reference will now be made, by way of example, to the accompanying drawings, in which:-

Figure 1 is a schematic sectional view through a laser dental handpiece,

Figures 2a and 2b are schematic views illustrating a laser focussing technique, and

Figure 3 illustrates how the handpiece can be angularly adjusted.

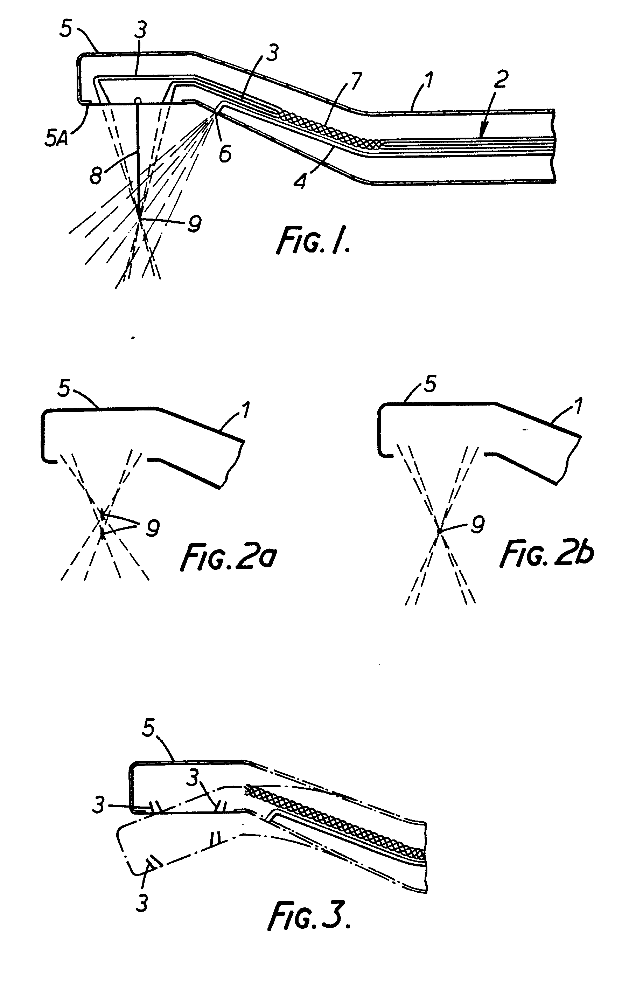

Referring firstly to Figure 1, the laser dental handpiece 1, of which only the working end is shown, includes a bundle 2 of optical fibres 3 through which laser beams can be transmitted, and a water/air supply tube 4.

The optical fibres 3 end in a working head 5 of the handpiece and the water/air tube 4 opens at 6 just below the head 5. The bundle 2 is spiralled at 7 to enable the head 5 to be pivoted relatively to the rest of the handpiece.

The head 5 also has provision for the removable insertion of a tactile probe 8. The head 5 may have a circular or oblong laser outlet face 5A.

The laser energy can be supplied by the domestic electrical supply or a generator/transformer unit.

The laser beam can then be broken down into smaller units or separate, smaller beams could be produced, which would then become a single powerful beam at the head 5. Of course, one laser generator could supply more than one dental handpiece at a time..

So far as the water and/or air supply is concerned, means may be provided to pulsate the water alternately with the laser beam if it is found that the laser will not function efficiently in a water-vapour atmosphere.

A further optical fibre could be provided to give a visual feedback to the operator of the operation being performed by the head 5.

The optical fibres 3 are arranged around the head 5, angled downwards and inwards towards a cutting focus point 9. It will be appreciated that the power of each individual laser from each optical fibre 3 would be harmless in itself but the combination of the laser beams focussed at 9 will be sufficient to produce the desired effect.

The handpiece can also be constructed to use single ray lasers which are sufficiently powerful to perform the required tasks at a predetermined distance from the head 5, beyond which distance the laser is rendered harmless. Such a construction can be operated with, or independently of, the aforementioned focussing system.

As illustrated in Figures 2a and b, it will be possible to focus the laser for different depths of tooth cavities, for example by arranging the focus 9 at different lengths from the head 5. By adjustment at the laser source, the focus 9 could in effect be de-focussed as shown in Figure 2a to give a cutting length of desired depth in order to produce a cut having a finite depth as opposed to a point.

As mentioned earlier, the head 5 can be pivotable to enable it to be suitably angled to reach areas of difficult access and, as shown in Figure 3, a locking system can be provided with parts of the handpiece being made flexible to facilitate the angular adjustment.

The tactile probe 8 is provided in order to give a physical effect for the operator and, because the focal lengths of the laser beams can be adjusted, different tactile probes of different lengths can be attached accordingly. In addition, the water/air outlet 6 can be adapted to spray water over the head 5 to clean it.

The laser dental handpiece can be used to perform various dental operations, e.g. to cut through tooth enamel, existing filling materials, dentine and caries and can also be used, by varying the laser power, to coagulate , cut or destroy soft tissues. For example, a reduced laser power can be used for gingival surgery, increased laser power for cutting (destroying) enamel, metal, etc. and intermediate laser power settings for trimming bone, etc. It will be appreciated that many variations are possible between the settings to provide a means for minor oral surgery of many kinds.

It is envisaged that it will also be possible to destroy caries from outside the tooth, provided that the extent of the caries was known, by using a small-bore hole in the side of the tooth to enable the insertion of an optical fibre connected to a screen to give a visual indication of the extent of the caries. It is envisaged that filling materials, as yet undeveloped, could then be used as "fillers" injected into the cavity made.

It will be appreciated that, compared with the known turbine-type dental handpieces, the present handpiece would be lightweight and highly manoeuvreable, providing easy access to the most difficult areas with a small working head. The handpiece would also be free from noise which could be a boon to the image of dentistry.

It is envisaged that such a handpiece could be adaptable to all forms of minor general surgery and possibly to industrial applications such as stone cutting, jewellery manufacture, micro-engineering, etc.

CLAIMS

1. A dental handpiece characterised in that it is arranged to perform dental work in the mouth by means of a laser beam.

2. A handpiece as claimed in claim 1, characterised by at least one optical fibre (3) through which a laser beam can be transmitted, the optical fibre ending in an outlet face (5A) of a head (5).

3. A handpiece as claimed in claim 2, characterised in that a plurality of said optical fibres (3) is provided in a bundle (2).

4. A handpiece as claimed in claim 3, characterised in that said bundle (2) is spiralled to enable the head (5) to be pivoted relatively to the rest of the handpiece.

5. A handpiece as claimed in claim 3 or 4, characterised in that said optical fibres (3) are arranged around said head (5) and are angled downwards and inwards towards a cutting focus point (9).

6. A handpiece as claimed in claim 5, characterised by means to adjust the focus point (9) and to de-focus it to give a cutting length of desired depth.

7. A handpiece as claimed in any one of claims 2 to 6, characterised in that said head (5) has a tactile probe (8).

8. A handpiece as claimed in any one of claims 2 to 7, characterised in that a water/air supply tube (4) is provided, said tube terminating at an outlet (6) of the handpiece.

9. A handpiece as claimed in any one of claims 2 to 8, characterised in that a further optical fibre is provided to give a visual feedback to the operator of the operation being performed.

10. A handpiece as claimed in any one of the preceeding claims, characterised by a laser source for the operation of the handpiece.

1/1

0073617

FiG. 1.

FiG. 2a

FiG. 2b

FiG. 3.

0073617

Application number

European Patent
Office

EUROPEAN SEARCH REPORT

EP 82 30 4404.5

| Category | Citation of document with indication, where appropriate, of relevant passages | Relevant to claim | CLASSIFICATION OF THE APPLICATION (Int. Cl. 3) |
|---|---|---|---|
| X | FR - A - 1 392 614 (PELLENC) <br> * abstract; fig. * | 1,2,6, 8,10 | A 61 C 1/08 <br><br> A 61 B 17/36 |
| X | DE - A1 - 2 828 322 (EICHLER et al.) <br> * claim 1 * | 1,2,6 | |
| A | GB - A - 1 417 906 (SHARON et al.) <br> * page 1, lines 10 to 71 * | 1,7 | |

**DOCUMENTS CONSIDERED TO BE RELEVANT**

TECHNICAL FIELDS SEARCHED (Int.Cl. 3)

A 61 B 17/00

A 61 C 1/00

A 61 C 3/00

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

The present search report has been drawn up for all claims

| Place of search | Date of completion of the search | Examiner |
|---|---|---|
| Berlin | 18-11-1982 | SIMON |

EPO Form 1503.1  06.78